Europäisches Patentamt

European Patent Office

Office européen des brevets

(11) Veröffentlichungsnummer: **0 192 084**
**B1**

## (12) EUROPÄISCHE PATENTSCHRIFT

(45) Veröffentlichungstag der Patentschrift:
02.11.88

(21) Anmeldenummer: **86101051.0**

(22) Anmeldetag: **27.01.86**

(51) Int. Cl.⁴: **G 01 N 27/56,** G 01 N 33/00

(54) Verfahren zur Messung des Sauerstoffgehaltes im Abgas von Brennkraftmaschinen.

(30) Priorität: **14.02.85 CH 670/85**

(43) Veröffentlichungstag der Anmeldung:
**27.08.86 Patentblatt 86/35**

(45) Bekanntmachung des Hinweises auf die Patenterteilung:
**02.11.88 Patentblatt 88/44**

(84) Benannte Vertragsstaaten:
**CH DE FR GB LI**

(56) Entgegenhaltungen:
**EP-A-0 028 826**
**EP-A-0 047 434**
**DE-A-3 316 854**
**FR-A-2 438 291**
**US-A-3 598 711**
**US-A-3 869 370**

(73) Patentinhaber: **BBC Brown Boveri AG,
Haselstrasse, CH- 5401 Baden (CH)**

(72) Erfinder: **Gautschi, Max, Dr., Neunbrunnenstrasse
249, CH- 8046 Zürich (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Messung des Sauerstoffgehalts im Abgas von Brennkraftmaschinen gemäss dem Oberbegriff des Anspruchs 1.

Zur Verbesserung des Abgasverhaltens von Brennkraftmaschinen werden in zunehmendem Masse Abgasfilter eingesetzt. Diese Filter halten in erster Linie Russpartikel zurück, können jedoch auch bei entsprechender katalytischer Reschichtung im Abgas vorhandene Schadstoffe katalytisch verbrennen. Diese Filter haben jedoch den Nachteil, dass sie im Laufe der Zeit durch den angesammelten Russ verstopfen, d.h. der Strömungswiderstand für das Abgas steigt extrem stark an. Man versucht deshalb, durch dauernde oder kurzzeitige Erhöhung der Filtertemperatur die Russbelegung zu verbrennen. Damit diese Verbrennung stattfinden kann, muss sichergestellt werden, dass im Abgas genügend Sauerstoff vorhanden ist. Zur Regelung des Sauerstoffgehalts beim Abbrennen und auch zum Erzeugen eines optimalen Brennstoffgemischs wird zwischen Motor und Abgasfilter als Sauerstoff-Sensor eine Lambda-Sonde eingebaut, deren Messignal einem Regelsystem der Brennkraftmaschine zugeführt wird, das in geeigneter Weise auf die Frischluftzufuhr und/oder die Treibstoffmenge einwirkt. Die üblicherweise verwendeten Lambdasonden weisen in ihrem Messkopf eine mit zwei Platinelektroden versehene Keramik auf, welche ein Ionenleiter für Sauerstoff ist. Ionenleiter für Sauerstoff sind z. B. die Keramikmaterialien $ZrO_2$, $TiO_2$, $LaF_3$, $SnO_2$, $Bi_2O_3$, $SrFeO_3$, $La_xSr_{1-x}CrO_3$. Bei einem unterschiedlichen Sauerstoffgehalt im Abgas und in der Umgebungsluft ergibt sich durch die Keramik im Messkopf der Lambda-Sonde eine Diffusion von Sauerstoffionen, was eine Spannungsdifferenz zwischen den beiden Platinelektroden zur Folge hat. Diese Spannungsdifferenz steht als Messignal zur Verfügung.

Eine zur Messung des Sauerstoffgehalts im Abgas von Brennkraftmaschinen relativ zum Sauerstoffgehalt der Luft geeignete Lambda-Sonde mit einer $ZrO_2$-Keramik ist aus einem Artikel von Hans-Martin Wiedenmann et al., "Heated Zirconia Oxygen Sensor for Stoichiometric and Lean Air-Fuel Ratios", SAE Paper 840141, SAE Congress, Detroit, Februar-März 1984 bekannt. Die bekannte Lambda-Sonde weist etwa die Form einer Zündkerze auf und ist direkt in eine mit einem entsprechenden Gewinde versehene Bohrung in einer Wand des Abgassystems einschraubbar. Im eingeschraubten Zustand ragt die den Messkopf der Lambda-Sonde bildende Keramik ein Stück weit in den Abgasraum hinein. Aus ihrem vorstehend bereits erläuterten Funktionsprinzip ergibt sich, dass das Messignal der bekannten Lambda-Sonde abhängig ist von den Sauerstoffpartialdrücken im Abgas und in der Luft. Der Sauerstoff-Partialdruck im Abgas ändert sich jedoch mit dem Abgasdruck. Nun ist der Druck des Abgases im Abgassystem einer Brennkraftmaschine keineswegs konstant, sondern hängt stark vom Grad der Verstopfung des Abgasfilters und der Motordrehzahl ab. Bei aufgeladenen Brennkraftmaschinen sind die Druckschwankungen im Abgassystem noch viel grösser, da sich zu den genannten Einflüssen Motordrehzahl und Verstopfungsgrad des Abgasfilters noch der jeweilige Aufladedruck addiert. Insgesamt kann der Druck des Abgases im Abgassystem um ein mehrfaches des Luftdrucks schwanken. Es versteht sich, dass unter solchen Umständen die Messung des prozentualen Sauerstoffgehalts im Abgas mittels der bekannten, direkt in eine Wand des Abgassystems eingeschraubten Lambda-Sonde keine brauchbaren Ergebnisse liefert. Der Einfluss des Abgasdrucks auf das Messignal der Lambda-Sonde könnte natürlich durch Verwendung eines Drucksensors und einer elektronischen Recheneinheit eliminiert werden. Dies ist jedoch eine aufwendige Lösung, da der Drucksensor im Abgassystem extrem korrosionsbeständig sein muss.

Die Erfindung, wie sie im Anspruch 1 gekennzeichnet ist, löst die Aufgabe, ein Verfahren zur Messung des Sauerstoffgehalts im Abgas von Brennkraftmaschinen mittels einer Lambda-Sonde anzugeben, bei welchem der Einfluss des Abgasdrucks auf das Messignal der Lambda-Sonde auf einfache Weise eliminiert ist.

Die Vorteile des erfindungsgemässen Verfahrens sind im wesentlichen darin zu sehen, dass das Messignal der Lambda-Sonde unabhängig vom Druck des Abgases im Abgassystem ist und dass dies ohne Messung des Drucks im Abgassystem und ohne elektronische Hilfsmittel erreicht wird.

Vorteilhafte Ausgestaltungen des erfindungsgemässen Verfahrens sind in den abhängigen Ansprüchen gekennzeichnet.

So lässt sich das Verfahren nach Anspruch 2 in besonders einfacher Weise technisch realisieren. Bei dieser einfachen Lösung könnte es jedoch vorkommen, dass das als Strömungswiderstand ausgebildete oder einen solchen enthaltende Rohr zum Messraum durch Russpartikel verstopft wird. Bei den Verfahrensvarianten nach Anspruch 4 und 5 kann dies durch die Verwendung von Ventilen nicht vorkommen.

Das erfindungsgemässe Verfahren weist in allen beanspruchten Ausbildungen den Vorteil auf, dass zur Messung des Sauerstoffgehalts im Abgas nur ein geringer Abgasanteil verwendet wird. Die darin enthaltene Schadstoffmenge ist derart unbedeutend dass sie an sich aus dem Messraum direkt ins Freie ausgestossen werden kann. Andererseits kann die zur Messung benötigte Abgasmenge auch dem Frischluftzufuhrsystem der Brennkraftmaschine zugeführt werden. In diesem Falle ergibt sich in vorteilhafter Weise überhaupt kein zusätzlicher Schadstoffausstoss an die Umgebungsluft.

Durch die Verwendung eines schlecht wärmeleitenden Materials für die Verbindung des Messraums mit dem Abgassystem lässt sich mit besonderem Vorteil zusätzlich noch der Einfluss der Temperaturschwankungen des Abgases auf das Messignal der Lambda-Sonde reduzieren. Dies ist vor allem bei Brennkraftmaschinen, die mit einem hohen Sauerstoffgehalt im Abgas gefahren werden, insbesondere bei Dieselmotoren, von ganz erheblicher Bedeutung.

Im folgenden werden Ausführungsbeispiele der Erfindung anhand der Zeichnung erläutert. Es zeigt:

Fig. 1 ein Blockschema einer Vorrichtung zur Durchführung des erfindungsgemässen Verfahrens mit kontinuierlicher Entnahme der Abgasprobemenge und

Fig. 2 ein Blockschema einer Vorrichtung zur Durchführung des erfindungsgemässen Verfahrens mit diskontinuierlicher Entnahme der Abgasprobemenge.

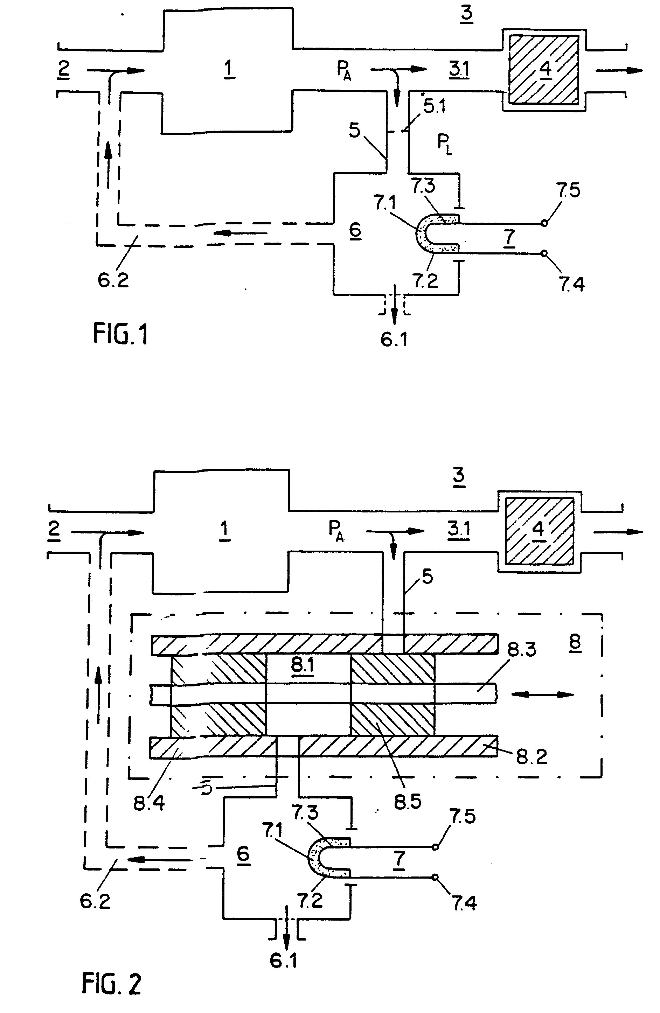

In Fig. 1 ist mit 1 eine Brennkraftmaschine bezeichnet, welche ihre Frischluft über ein Frischluftzuführsystem 2 erhält. Das Abgas der Brennkraftmaschine 1 entweicht über ein Abgassystem 3 in welchem ein Russfilter 4 angeordnet ist. Das Russfilter 4 stellt insbesondere nach längerem Gebrauch und starker Verstopfung mit Russpartikeln einen erheblichen Strömungswiderstand für das Abgas der Brennkraftmaschine 1 dar, so dass sich im Abgassystem 3 zwischen der Brennkraftmaschine 1 und dem Russfilter 4 ein gegenüber dem äusseren Luftdruck $P_L$ erhöhter Druck $P_A$ im Abgas einstellt. Mit dem mit 3.1 bezeichneten unter Überdruck stehenden Teil des Abgassystems 3 steht über ein Rohr 5 ein Messraum 6 in Verbindung, welcher entweder über eine Öffnung 6.1 zur Umgebungsluft hin offen oder über ein Rohr 6.2 mit dem Frischluftzuführsystem 2 in Verbindung steht. In einer Wand des Messraums 6 ist eine rein schematisch dargestellte Lambda-Sonde 7 angeordnet. Der Messkopf dieser Lambda-Sonde 7 besteht aus einem Festkörperelektrolyten 7.1, welcher auf seinen Oberflächen mit Platinelektroden 7.2 und 7.3 versehen ist. Die Platinelektrode 7.2 steht ausschliesslich mit dem Gas im Messraum 6 und die Platinelektrode 7.3 ausschliesslich mit der Umgebungsluft in Kontakt. Die Platinelektroden 7.2 und 7.3 sind schliesslich mit Anschlusskontakten oder Anschlussklemmen 7.4 und 7.5 verbunden, an welchen die Meßspannung oder Meßstrom der Lambda-Sonde 7 abgegriffen werden kann. Als Material für den Festkörperelektrolyten 7.1 kann $ZrO_2$, $TiO_2$, $LaF_3$, $SnO_2$, $Bi_2O_3$, $SrFeO_3$ oder $La_xSr_{1-x}CroO_3$ verwendet werden. Bevorzugt kommt jedoch die zuerst genannte Zirkonoxydkeramik in Betracht.

Bei der Vorrichtung nach Fig. 1 wird ein Teil des Abgases aus dem unter Überdruck stehenden Teil 3.1 des Abgassystems 3 über das Rohr 5 in den Messraum 6 kontinuierlich einströmen und sich dabei auf einen Druck entspannen, der, bedingt durch die Öffnung 6.1 oder alternativ das Rohr 6.2 zum Frischluftzuführsystem 2, in etwa dem äusseren Luftdruck $P_L$ entspricht. Die Lambda-Sonde 7 ermittelt den Sauerstoffgehalt im Abgas daher stets unter gleichbleibenden Druckverhältnissen. Die über das Rohr 5 in den Messraum 6 fliessende Abgasmenge hängt ab vom Strömungswiderstand des Rohres 5, welcher über seinen Querschnitt und seine Länge oder durch eingebaute Blenden 5.1 oder poröse Keramikkörper kontrollierbar ist. Die zur Messung mit der Lambda-Sonde 7 benötigte Abgasmenge dürfte in praktisch allen Anwendungsfällen so klein sein, dass sie über die Öffnung 6.1 ohne weiteres an die Umgebungsluft abgegeben werden kann, ohne dass daraus eine nennenswerte Schadstoffbelastung der Umgebungsluft resultiert. Will man dennoch die Abgabe ungereinigten Abgases an die Umgebungsluft vollständig vermeiden, so kann die zur Messung benötigte Abgasmenge über das Rohr 6.2 der Brennkraftmaschine 1 über ihr Frischluftzuführsystem 2 wieder zugeführt werden.

Die Vorrichtung nach Fig. 2 entspricht bis auf die zusätzlich im Rohr 5 vorgesehene Ventilanordnung 8 der Vorrichtung nach Fig. 1. Die Ventilanordnung 8 dient zur Dosierung der Abgasprobemenge. Im Beispiel von Fig. 1 wird diese Dosierung, wie beschrieben, über den Strömungswiderstand des Rohres 5 bei kontinuierlicher Strömung erreicht. Ist die zur Messung benötigte Abgasprobemenge jedoch klein, so müssten im Beispiel von Fig. 1 entsprechend kleine Strömungsquerschnitte im Rohr 5 vorgesehen werden. Dabei besteht die Gefahr einer Verstopfung durch Russpartikel. Die in Fig. 2 vorgesehene Ventilanordnung 8 erlaubt nun, grössere Querschnitte im Rohr 5 zu verwenden.

Besteht die Ventilanordnung 8, wie in Fig. 2 nicht dargestellt, aus einem einzigen Ventil, so hängt die Menge des in den Messraum 6 strömenden Abgases von der Öffnungsdauer dieses Ventils ab.

Wird dagegen eine Ventilanordnung 8 verwendet, wie sie in Fig. 2 in einem Schnitt dargestellt ist, welche zwei, einen Zwischenraum 8.1 begrenzende, jeweils gegensinnig geöffnete bzw. geschlossene Ventile aufweist, so ist die Abgasprobemenge durch die Grösse des Zwischenraumes 8.1 bestimmt, zumindest dann, wenn die Ventile jeweils lange genug geöffnet sind, um einen vollständigen Druckausgleich zwischen dem Teil 3.1 des Abgassystems 3 und dem Zwischenraum 8.1 und zwischen diesem und dem Messraum 6 zu ermöglichen.

Die in Fig. 2 dargestellte Ventilanordnung 8 besteht einfach aus einem Zylinder 8.2 in welchem zwei über eine Stange 8.3 starr miteinander verbundene Kolben 8.4 und 8.5 beweglich sind. Der Zylinder 8.2 und die Kolben 8.4 und 8.5 begrenzen den Zwischenraum 8.1. Der Zylinder 8.2 weist in seiner Wand zwei Öffnungen zum Anschluss des Rohres 5 auf. Diese Öffnungen sind bezüglich der Längsachse des Zylinders 8.2 gegeneinander versetzt. Durch Verschieben der beiden Kolben 8.4 und 8.5 kann der Zwischenraum 8.1 alternativ zum Teil 3.1 des Abgassystems 3 und zum Messraum 6 hin geöffnet werden. Die Stange 8.3 könnte über ein Pleuel beispielsweise von der Nockenwelle der Brennkraftmaschine 1 angetrieben werden.

Durch die Verwendung der Ventilanordnung 8 erfolgt die Entnahme der Abgasprobemenge aus dem Abgassystem 3 diskontinuierlich.

Bei Lambda-Sonden 7 der hier verwendeten Art ist die Diffusion des Sauerstoffs durch den Festkörperelektrolyten 7.1 und damit auch das Messignal stark temperaturabhängig. Zur Erzielung eines guten Messergebnisses ist es daher erforderlich, die Temperatur des Festkörperelektrolyten 7.1, z. B. mittels einer in der Lambda-Sonde integrierten Heizung, auf einem Wert an der Obergrenze der vorkommenden Abgastemperaturen konstant zu halten. Durch Verwendung eines schlecht wärmeleitenden Materials für die Verbindung zwischen Abgassystem 3 und Messraum 6, also besonders für das Rohr 5, lässt sich diese Forderung wesentlich leichter erfüllen. Eine ausreichend geringe Wärmeleitfähigkeit weist z. B. nichtrostender Stahl auf. Durch diese Massnahme wird in vorteilhafter Weise eine weitere Verbesserung der Messung des Sauerstoffgehalts im Abgas erreicht.

**Patentansprüche**

1. Verfahren zur Messung des Sauerstoffgehalts im Abgas von Brennkraftmaschinen (1) relativ zum Sauerstoffgehalt der Luft in einem bezüglich des Luftdrucks ($P_L$) unter Überdruck stehenden Teil (3.1) des Abgassystems (3) mittels einer auf dem Prinzip der Sauerstoffdiffusion in Festkörperelektrolyten (7.1) basierenden Lambda-Sonde (7), dadurch gekennzeichnet, dass aus dem unter Überdruck stehenden Teil (3.1) des Abgassystems (3) eine Abgasprobemenge entnommen und nach Entspannung auf den äußeren Luftdruck ($P_L$) der Lambda-Sonde (7) zugeführt wird.

2. Verfahren nach Anspruch 1, dadurch gekennzeichnet, dass die Abgasprobemenge aus dem unter Überdruck stehenden Teil (3.1) des Abgassystems (3) durch eine kontinuierliche Strömung über ein, einen Strömungswiderstand bildendes oder einen solchen enthaltendes Rohr (5) in einen zur Umgebungsluft oder zum Frischluftzuführsystem (2) der Brennkraftmaschine (1) offenen Messraum (6) entnommen und entspannt wird, in dessen Wand der Festkörperelektrolyt (7.1) der Lambda-Sonde (7) angeordnet ist.

3. Verfahren nach Anspruch 2, dadurch gekennzeichnet, dass als Strömungswiderstand im Rohr (5) eine Blende (5.1) oder ein poröser Keramikkörper verwendet wird.

4. Verfahren nach Anspruch 1, dadurch gekennzeichnet, dass die Abgasprobemenge aus dem unter Überdruck stehenden Teil (3.1) des Abgassystems (3) durch kurzzeitiges Öffnen eines Ventils (9) diskontinuierlich in einen zur Umgebungsluft oder zum Frischluftzuführsystem (2) der Brennkraftmaschine (1) offenen Messraum (6) entnommen und entspannt wird, in dessen Wand der Festkörperelektrolyt (7.1) der Lambda-Sonde (7) angeordnet ist.

5. Verfahren nach Anspruch 1, dadurch gekennzeichnet, dass die Abgasprobemenge aus dem unter Überdruck stehendenTeil (3.1) des Abgassystems (3) durch kurzzeitiges Öffnen eines ersten Ventils in einen geschlossenen Zwischenraum (8.1) und nach dem Schliessen des ersten Ventils durch Öffnen eines zweiten Ventils in einen zur Umgebungsluft oder zum Frischluftzuführsystem (2) der Brennkraftmaschine (1) offenen Messraum (6) hinein entspannt wird, in dessen Wand der Festkörperelektrolyt (7.1) der Lambda-Sonde (7) angeordnet ist.

6. Verfahren nach Anspruch 5, dadurch gekennzeichnet, dass der Zwischenraum (8.1) durch zwei starr miteinander verbundene Kolben (8.4, 8.5) in einem Zylinder (8.2) gebildet wird und durch Verschieben der Kolben (8.4, 8.5) im Zylinder (8.2) entweder zum Abgassystem (3) oder dem Messraum (6) hin geöffnet bzw. verschlossen wird.

7. Verfahren nach Anspruch 6, dadurch gekennzeichnet, dass die Kolben (8.4, 8.5) von der Nockenwelle der Brennkraftmaschine (1) angetrieben werden.

**Claims**

1. Process for measuring the oxygen content in the exhaust of internal-combustion engines (1) relative to the oxygen content of the air in a part (3.1) of the exhaust system (3) under above-atmospheric pressure with respect to the air pressure ($P_A$) by means of a lambda probe (7) based on the principle of oxygen diffusion in solid-state electrolytes (7.1), characterized in that an amount of exhaust sample is taken from the part (3.1) of the exhaust system (3) under above-atmospheric pressure and, after expansion to the external air pressure ($P_A$), is fed to the lambda probe (7).

2. Process according to claim 1, characterized in that the amount of exhaust sample is taken from the part (3.1) of the exhaust system (3) under above-atmospheric pressure by a continuous flow via a pipe (5) forming a flow resistance, or containing such a flow resistance, and expanded into a measuring chamber which is open to the surrounding air or to the fresh-air supply system (2) of the internal-combustion engine (1) and in the wall of which the solid-state electrolyte (7.1) of the lambda probe (7) is arranged.

3. Process according to claim 2, characterized in that a baffle (5.1) or a porous ceramic element is used as flow resistance in the pipe (5).

4. Process according to claim 1, characterized in that the amount of exhaust sample is taken from the part (3.1) of the exhaust system (3) under above-atmospheric pressure by brief opening of a valve (8) discontinuously and expanded into a measuring chamber (6) which is open to the surrounding air or to the fresh-air supply system (2) of the internal-combustion engine (1) and in the wall of which the solid-state electrolyte (7.1) of the lambda probe (7) is arranged.

5. Process according to claim 1, characterized in that the amount of exhaust sample is taken from the part (3.1) of the exhaust system (3) under above-atmospheric pressure by brief opening of a first valve and expanded into an enclosed intermediate space (8.1) and, after closing the first valve, by opening a second valve it is expanded into a measuring chamber (6) which is open to the surrounding air or to the fresh-air supply system (2) of the internal-combustion engine (1) and in the wall of which the solid-state electrolyte (7.1) of the lambda probe (7) is arranged.

6. Process according to claim 5, characterized in that the intermediate space (8.1) is formed by two plungers (8.4, 8.5) rigidly connected to each other in a cylinder (8.2) and is opened or closed relative to either the exhaust system (3) or the measuring chamber (6) by displacement of the plungers (8.4, 8.5) in the cylinder (8.2).

7. Process according to claim 6, characterized in that the plungers (8.4, 8.5) are driven by the camshaft of the internal-combustion engine (1).

## Revendications

1. Procédé pour la mesure de la concentration en oxygène des gaz d'échappement de moteurs à combustion (1) par rapport à la teneur en oxygène de l'air, dans une partie (3.1) du système d'échappement (3) sous surpression par rapport à la pression de l'air (P$_L$), au moyen d'une sonde Lambda (7) basée sur le principe de la diffusion de l'oxygène dans un électrolyte solide (7.1), caractérisé en ce qu'un échantillon de gaz d'échappement est prélevé dans la partie sous surpression (3.1) du système d'échappement (3) et est amené à la sonde Lambda (7) après détente jusqu'à la pression de l'air extérieur (P$_L$).

2. Procédé suivant la revendication 1, caractérisé en ce que l'échantillon de gaz d'échappement est prélevé dans la partie sous surpression (3.1) du système d'échappement et est détendu par un écoulement continu par un tube (5) constituant une résistance à l'écoulement ou contenant une telle résistance, dans une chambre de mesure communiquant avec l'air extérieur ou avec le système d'alimentation en air frais (2) du moteur à combustion (1) et dans la paroi de laquelle est agence l'électrolyte solide (7.1) de la sonde Lambda (7).

3. Procédé suivant la revendication 2, caractérisé en ce qu'une chicane (5.1) ou un corps en céramique poreux est utilisé comme résistance à l'écoulement dans le tube (5).

4. Procédé suivant la revendication 1, caractérisé en ce que l'échantillon de gaz d'échappement est prélevé de façon discontinue dans la partie sous surpression (3.1) du système d'échappement (3) et est détendu par l'ouverture brève d'une valve (8) dans une chambre de mesure (6) communiquant avec l'air extérieur ou avec le système d'alimentation en air frais (2) du moteur à combustion et dans la paroi de laquelle est agencé l'électrolyte solide (7.1) de la sonde Lambda (7).

5. Procédé suivant la revendication 1, caractérisé en ce que l'échantillon de gaz d'échappement de la partie sous surpression (3.1) du système d'échappement (3) est détendu par la brève ouverture d'une première valve dans une chambre intermédiaire fermée (8.1) et, après fermeture de la première valve, par ouverture d'une seconde valve dans une chambre de mesure (6) communiquant avec l'air extérieur ou avec le système d'alimentation en air frais (2) du moteur à combustion (1) et dans la paroi de laquelle est agencé l'électrolyte solide (7.1) de la sonde Lambda (7).

6. Procédé suivant la revendication 5, caractérisé en ce que la chambre intermédiaire (8.1) est définie par deux pistons (8.4, 8.5) reliés rigidement l'un à l'autre dans un cylindre (8.2) et peut être ouverte ou fermée vers le système d'échappement (3) ou vers la chambre de mesure (6) par le coulissement des pistons (8.4, 8.5) dans le cylindre (8.2).

7. Procédé suivant la revendication 6, caractérisé en ce que les pistons (8.4, 8.5) sont actionnés par l'arbre à cames du moteur à combustion (1).

FIG.1

FIG. 2

1